# EUROPEAN PATENT APPLICATION

(11) **EP 4 796 152 A1**
(43) Date of publication of application: **26.08.2026**
(21) Application number: 24878764.0
(22) Date of filing: 14.09.2024
(51) Int. Cl.: A61M 16/01, A61M 16/00

(54) **CONTINUOUS RECIPROCATING DRUG DELIVERY DEVICE**

(30) Priority: 16.10.2023 CN 202311334854
(71) Applicant: Beijing Aeonmed Co., Ltd., Beijing 100070 (CN)
(72) Inventor: WANG, Zhimeng, Beijing 100070 (CN); CHEN, Wansheng, Beijing 100070 (CN); LIU, Jialong, Beijing 100070 (CN); DUAN, Jinmin, Beijing 100070 (CN); LI, Zongyang, Beijing 100070 (CN)
(74) Representative: Bryn Aarflot AS
(86) International application number: PCT/CN2024/118970
(87) International publication number: WO 2025/082115

(57) **Abstract**

A continuous reciprocating drug delivery device includes a plunger pump mechanism, an upper valve plate (27), a lower valve plate (28), and a second solenoid valve (35). The plunger pump mechanism includes an upper plunger chamber and a lower plunger chamber. When a plunger rod (30) moves upward, a volume of the upper plunger chamber decreases and a volume of the lower plunger chamber increases. When the plunger rod (30) moves downward, the volume of the lower plunger chamber decreases and the volume of the upper plunger chamber increases. The upper valve plate (27) and the lower valve plate (28) jointly provide fluid channels separately between a tank body (3) and an upper plunger chamber upper opening, between the upper plunger chamber upper opening and an upper plunger chamber lower opening, between the upper plunger chamber lower opening and an evaporation chamber of an evaporation mechanism, between the tank body (3) and a lower plunger chamber upper opening, between the lower plunger chamber upper opening and a lower plunger chamber lower opening, and/or between the lower plunger chamber lower opening and the evaporation chamber of the evaporation mechanism. The lower plunger chamber upper opening is further selectively in communication with the tank body (3) through the second solenoid valve (35). The drug delivery device has a simple structure, which is conducive to processing and easy for productization.

## Description

### CROSS REFERENCE TO RELATED APPLICATION

The present application claims priority to Chinese Patent Application No. 202311334854.9, filed on October 16, 2023, and entitled "CONTINUOUS RECIPROCATING DRUG DELIVERY DEVICE", which is incorporated herein by reference in its entirety.

### TECHNICAL FIELD

The present application relates to the technical field of anesthesia equipment, and in particular, to a continuous reciprocating drug delivery device.

### BACKGROUND

Vaporizers used in medical anesthesia machines can be roughly divided into two categories: One category is traditional mechanical vaporizers. These vaporizers have gone through a relatively long development process with the advancement of anesthesia technology, and are still used in various low-end anesthesia machines at present. Typical representatives of this type of vaporizers include variable bypass in-circuit vaporizers, flow-measuring general quantitative out-of-circuit vaporizers, variable bypass dedicated quantitative out-of-circuit vaporizers, and injection vaporizers, etc. The other category is electronically controlled vaporizers. Based on the characteristics of anesthetic drugs and the principle of mechanical vaporizers, these vaporizers integrate the features of electronic control technology and measurement technology, resulting in significant improvements of vaporizers in aspects such as concentration control and compliance. Typical representatives in this regard include the Aladin 2222 electronically controlled vaporizer, the Tec6 desflurane vaporizer, the GE/Datex-Ohmeda Aladin and Aladin 2 cartridge vaporizers, as well as the Maquet FLOW-i and Drager DIVA electro-spray vaporizers.

The core point of an anesthesia vaporizer is to be able to control the precise output and rapid response of the anesthetic concentration. Analyzing the characteristics of the above-mentioned vaporizers, it can be seen that except for the Maquet FLOW-i and Drager DIVA electro-spray vaporizers, whether they are traditional mechanical vaporizers or electronically controlled vaporizers, all of the other vaporizers first evaporate the liquid anesthetic into a gaseous state through various methods, and then control the output ratio of the anesthetic gas to be proportioned with a fresh gas to achieve an output at a definite concentration. This solution has a very big drawback. Since gases are affected by temperature and ambient pressure, it is very difficult to precisely control the amount of substance, which leads to a large error in the anesthetic concentration output by the vaporizer.

Compared with other vaporizers, the Maquet FLOW-i and Drager DIVA electro-spray vaporizers control the dosage of the liquid anesthetic sprayed. After being sprayed, the anesthetic is fully evaporated in the evaporation chamber through a method such as heating and atomization, and then mixed with a fresh gas to achieve concentration control. Although the electro-spray vaporizers control the dosage of the liquid drug, they are powered by compressed air. It is difficult to control the amount of anesthetic sprayed. Therefore, it is very difficult to precisely control the anesthetic concentration. A relatively high-precision concentration can be obtained only by intermittently spraying small amounts and taking an average value.

The patent application with Application No. 202211660947.6 discloses an electronically controlled vaporizer and a control method thereof, proposing control of the volume of anesthetic by controlling the pushing speed through a plunger pump system and a valve seat, thereby improving the accuracy of the output anesthetic concentration by utilizing the incompressibility of liquid volume. Specifically, the plunger pump divides the plunger cavity into left and right cavities through a piston. The volume of the anesthetic is controlled by controlling the extension and retraction velocity of the piston through the plunger pump. If the pushed liquid anesthetic is completely evaporated after being mixed with a fresh gas, an anesthetic-mixed gas with a certain concentration can be obtained. However, the plunger pump in the present application is of the piston type. The piston divides the plunger pump cavity into two cavities. To achieve the function of exhausting gas bubbles, it can only be placed horizontally, which is not conducive to product miniaturization. Moreover, the inner hole of the plunger pump cavity needs to be finely machined, and the surface roughness needs to be very high, resulting in high processing costs. In addition, the present application is selectively in communication with the plunger cavity and the evaporation cavity through a switching valve and the upper and lower valve plates of the evaporation core. However, using two switching valves results in high processing costs, difficult assembly, and a large number of points that need to be sealed. In summary, this patent application has the disadvantages of being unfavorable for product miniaturization, having high processing costs, high sealing difficulty, a complex structure, and being unfavorable for production during the processing and production process.

### SUMMARY OF THE INVENTION

The object of the present application is to provide a continuous reciprocating drug delivery device to overcome the problems of the existing drug delivery devices, such as being unfavorable for product miniaturization, having high processing costs, great sealing difficulties, complex structures, and being unfavorable for production.

To solve the above technical problems, the technical solution of the present application provides a continuous reciprocating drug delivery device, including: a drug storage tank mechanism, configured to hold anesthetic liquid 20; and an evaporation mechanism, configured to evaporate the anesthetic liquid 20 into a gaseous state to be proportioned with a fresh gas to output a mixed anesthetic gas at a predetermined concentration, where the device further includes a plunger pump mechanism, an upper valve plate 27, a lower valve plate 28, and a second solenoid valve 35, where
the evaporation mechanism includes an evaporation seat 16, and the evaporation seat 16 is vertically provided with a deep-hole cavity for use as a total plunger chamber of the plunger pump mechanism;
a sealing sleeve 33 is disposed inside the total plunger chamber and divides the total plunger chamber into an upper plunger chamber and a lower plunger chamber, an upper plunger chamber upper opening and an upper plunger chamber lower opening are provided in a sidewall of the upper plunger chamber, and a lower plunger chamber upper opening and a lower plunger chamber lower opening are provided in a sidewall of the lower plunger chamber;
the plunger pump mechanism is further provided with a plunger rod 30, the plunger rod 30 is connected to an output shaft of a linear motor 11 and moves up and down in the upper plunger chamber and the lower plunger chamber under control of the linear motor 11, the plunger rod 30 includes a plunger rod lower section and a plunger rod upper section, a cross-sectional area of the plunger rod lower section is larger than a cross-sectional area of the plunger rod upper section, a length of the plunger rod lower section is greater than a height of the lower plunger chamber, a bottommost end of the plunger rod lower section passes through the sealing sleeve 33 and remains in the lower plunger chamber, when the plunger rod 30 moves upward, a volume of the upper plunger chamber decreases and a volume of the lower plunger chamber increases, and when the plunger rod 30 moves downward, the volume of the lower plunger chamber decreases and the volume of the upper plunger chamber increases;
the upper valve plate 27 and the lower valve plate 28 are in close contact to form a switching valve, the upper valve plate 27 is driven by a rotary motor 12 to rotate, the upper valve plate 27 and the lower valve plate 28 jointly provide fluid channels separately between a tank body 3 of the drug storage tank mechanism and the upper plunger chamber upper opening, between the upper plunger chamber upper opening and the upper plunger chamber lower opening, between the upper plunger chamber lower opening and an evaporation chamber of the evaporation mechanism, between the tank body 3 and the lower plunger chamber upper opening, between the lower plunger chamber upper opening and the lower plunger chamber lower opening, and/or between the lower plunger chamber lower opening and the evaporation chamber of the evaporation mechanism; and
the lower plunger chamber upper opening is further selectively in communication with the tank body 3 through the second solenoid valve 35.

As an improvement of the above device, an outer ring of the sealing sleeve 33 is fixedly connected to an inner wall of the total plunger chamber through a sealing ring, and an inner ring is in close contact with the plunger rod 30 through a sealing ring; and the linear motor 11 is equipped with a first encoder 10 configured to feed back a movement distance of the output shaft of the linear motor 11 in real time to achieve precise control of movement of the plunger rod 30.

As an improvement of the above device, an upper end of the evaporation seat 16 is provided with a circular edge-cut groove, a bottom of the groove is provided with a first flat airway port 36 and a second flat airway port 37, the first flat airway port 36 is in communication with a fresh gas inlet 29, the second flat airway port 37 is in communication with a mixed anesthetic gas outlet 32, an evaporation seat boss is disposed between the first flat airway port 36 and the second flat airway port 37, and a hole X'1, a hole X'2, a hole X'3, a hole X'4, and a hole X'5 are circumferentially distributed near an inner edge of the bottom of the circular edge-cut groove, where
the hole X'1 is connected to the tank body 3 of the drug storage tank mechanism through a first internal pipe of the evaporation seat 16;
the hole X'2 is in communication with the upper plunger chamber upper opening through a second internal pipe of the evaporation seat 16;
the hole X'3 is in communication with the upper plunger chamber lower opening through a third internal pipe of the evaporation seat 16;
the hole X'4 is in communication with the lower plunger chamber upper opening through a fourth internal pipe of the evaporation seat 16;
the hole X'5 is in communication with the lower plunger chamber lower opening through a fifth internal pipe of the evaporation seat 16;
the lower valve plate 28 is provided with a circular edge-cut boss corresponding to the circular edge-cut groove of the evaporation seat 16 for blind insertion and positioning between the lower valve plate 28 and the evaporation seat 16, the circular edge-cut boss is further provided with a hole X1, a hole X2, a hole X3, a hole X4, and a hole X5 that correspond one-to-one to the hole X'1, the hole X'2, the hole X'3, the hole X'4, and the hole X'5, respectively, the lower valve plate 28 is provided with a lower valve plate groove, the lower valve plate groove covers the first flat airway port 36, the second flat airway port 37, and the evaporation seat boss, and a gap exists between a bottom surface of the lower valve plate groove and a top surface of the evaporation seat boss, the gap between the bottom surface of the lower valve plate groove and the top surface of the evaporation seat boss is used as the evaporation chamber, and a plurality of through holes are further arranged in an arc at a bottom of the lower valve plate groove; and
the upper valve plate 27 is driven by the rotary motor 12 to rotate, the upper valve plate 27 is symmetrically provided with a first I-shaped groove and a second I-shaped groove, an inner-layer groove and an outer-layer groove of each of the first I-shaped groove and the second I-shaped groove are both arc-shaped, and centers of the inner-layer groove and the outer-layer groove are connected by a straight groove, radians of the outer-layer grooves of the first I-shaped groove and the second I-shaped groove are the same as circumferential radians between the hole X1, the hole X2, the hole X3, the hole X4, and the hole X5, and radians of the inner-layer grooves of the first I-shaped groove and the second I-shaped groove are the same as an arrangement radian of the through holes.

As an improvement of the above device, when the outer-layer groove of the first I-shaped groove covers the hole X1 and the hole X2, the first I-shaped groove provides a fluid channel between the hole X1 and the hole X2, and the anesthetic liquid 20 flows from the tank body 3 of the drug storage tank mechanism into the upper plunger chamber of a first plunger pump set sequentially through the hole Y1, the first internal pipe of the evaporation seat 16, the hole X'1, the hole X1, the first I-shaped groove, the hole X2, the hole X'2, the second internal pipe of the evaporation seat 16, and the upper plunger chamber upper opening;
when the outer-layer groove of the first I-shaped groove covers the hole X2 and the hole X3, the first I-shaped groove provides a fluid channel between the hole X2 and the hole X3;
when the outer-layer groove of the first I-shaped groove covers the hole X3 and the inner-layer groove of the first I-shaped groove covers the through holes of the lower valve plate 28, the first I-shaped groove provides a fluid channel between the hole X3 and the through holes, and the anesthetic liquid 20 in the upper plunger chamber flows into the evaporation chamber sequentially through the upper plunger chamber lower opening, the third internal pipe of the evaporation seat 16, the hole X'3, the hole X3, the first I-shaped groove, and the through holes;
when the outer-layer groove of the second I-shaped groove covers the hole X1 and the hole X4, the second I-shaped groove provides a fluid channel between the hole X1 and the hole X4, and the anesthetic liquid 20 flows from the tank body 3 of the drug storage tank mechanism into the lower plunger chamber sequentially through the hole Y1, the first internal pipe of the evaporation seat 16, the hole X'1, the hole X1, the second I-shaped groove, the hole X4, the hole X'4, the fourth internal pipe of the evaporation seat 16, and the lower plunger chamber upper opening;
when the outer-layer groove of the second I-shaped groove covers the hole X4 and the hole X5, the second I-shaped groove provides a fluid channel between the hole X4 and the hole X5; and
when the outer-layer groove of the second I-shaped groove covers the hole X5 and the inner-layer groove of the second I-shaped groove covers the through holes of the lower valve plate 28, the second I-shaped groove provides a fluid channel between the hole X5 and the through holes, and the anesthetic liquid 20 in the lower plunger chamber flows into the evaporation chamber sequentially through the lower plunger chamber lower opening, the fifth internal pipe of the evaporation seat 16, the hole X'5, the hole X5, the second I-shaped groove, and the through holes.

As an improvement of the above device, the first flat airway port 36 is in communication with the fresh gas inlet 29, and the second flat airway port 37 is connected to the mixed anesthetic gas outlet 32; and the anesthetic liquid 20 in the upper plunger chamber or the lower plunger chamber flows into the gap between the lower valve plate groove and the evaporation seat boss through the through holes and evaporates, and the evaporated anesthetic gas and the fresh gas flowing in through the fresh gas inlet 29 are mixed and output through the mixed anesthetic gas outlet 32.

As an improvement of the above device, a concentration regulator 31 is disposed between the second flat airway port 37 and the mixed anesthetic gas outlet 32 and configured to adjust a cross-sectional area of a fluid channel between the second flat airway port 37 and the mixed anesthetic gas outlet 32.

As an improvement of the above device, the upper valve plate 27, a thrust bearing 26, a connecting shaft 25, a spring, a rotary motor connecting seat 14, the rotary motor 12, and a second encoder are sequentially disposed above the lower valve plate 28, where
the spring is clamped between the rotary motor connecting seat 14 and the thrust bearing 26, a compression force of the spring is sequentially transmitted to the thrust bearing 26, the upper valve plate 27, and the lower valve plate 28 to enable the upper valve plate 27 to be in close contact with the lower valve plate 28;
the upper valve plate 27 is coupled to the connecting shaft 25, and a motor shaft of the rotary motor drives the upper valve plate 27 to rotate through the connecting shaft 25 to achieve transmission of torque; and
the second encoder is configured to position a rotation angle of the upper valve plate 27.

As an improvement of the above device, the drug storage tank mechanism includes the tank body 3, a liquid level column 2, an electronic liquid level gauge 9, a pressure sensor, and a first solenoid valve 8, where
the tank body 3 includes a drug injection port 5 configured to inject the anesthetic liquid 20 and a drug discharge port configured to discharge the anesthetic liquid 20 outside, the drug injection port 5 is provided with a drug cap 6, and opening and closing of the drug discharge port are controlled by a drug discharge knob 17;
the liquid level column 2 is in communication with the tank body 3 and configured to manually observe a liquid level height of the tank body 3;
the electronic liquid level gauge 9 is configured to monitor the liquid level height of the tank body 3 in real time;
the pressure sensor is configured to monitor pressure inside the tank body 3 in real time; and
the first solenoid valve 8 is configured to control communication between the tank body 3 and a fresh gas inlet 29.

The present application includes the following advantages:
1. The present application realizes the precise control and rapid response of anesthetic concentration. The electronically controlled vaporizer provided by the present application controls the volume of the anesthetic by controlling the pushing speed of the anesthetic liquid 20, thereby improving the accuracy of the output anesthetic concentration by utilizing the incompressibility of liquid volume. The influence of temperature, air pressure, etc. on the liquid anesthetic can be ignored. The linear motor is used to precisely control the drug-pushing speed of the plunger pump. The output anesthetic is immediately evaporated after being mixed with fresh gas, and therefore a definite anesthetic concentration is obtained. If different concentrations of anesthetic gas at different flow rates are desired, it is only necessary to control the flow rate of the fresh gas and the drug-pushing speed of the plunger pump.
2. The present application only uses one switching valve. The enablement and disablement of flow at the drug inlets and outlets of the two plunger pump assemblies are controlled by controlling the rotation angle of the switching valve in combination with the on-off of the second solenoid valve, so as to realize the drug suction and drug pushing functions of the plunger pumps. When one plunger pump assembly is sucking in drug, the other plunger pump assembly can push out drug simultaneously. Moreover, using only one switching valve results in a small assembly difficulty, fewer points to be sealed, and low costs. Therefore, the structure is simple, which is conducive to processing and production and easy for productization.
3. The present application utilizes a plunger-type plunger pump mechanism, which enables the plunger pump mechanism to be placed vertically, and the plunger chamber is located in the evaporation seat. This is beneficial for product miniaturization. Moreover, the overall structural form is more compact, the weight is lighter, the installation of pipes is reduced, and the reliability is greatly improved.
4. Compared with the piston structure adopted by the existing drug delivery devices, which requires fine machining of the inner hole of the plunger pump cavity, the present application adopts a plunger-type plunger pump mechanism, which performs fine machining on the plunger rod. The outer surface roughness of the plunger rod is required to be relatively high. However, the outer surface of the plunger rod is an outer circle, which is very easy to perform fine machining on, and the processing costs are low.
5. The concentration regulator 31 is installed between the O₂ flat airway hole in the evaporation seat 16 and the mixed anesthetic gas outlet 32. The cross-sectional area of the fluid channel between the second flat airway port 37 and the mixed anesthetic gas outlet 32 can be adjusted by adjusting the concentration regulator 31. As the concentration regulator 31 squeezes the fluid channel, the cross-sectional area of the fluid channel decreases. The concentration regulator 31 adjusts the flow velocity in the fluid channel by adjusting the cross-sectional area of the fluid channel. When the cross-sectional area decreases, that is, after the fluid channel becomes narrow, the flow velocity increases again. If the anesthetic liquid 20 flowing out of the small holes of the lower valve plate 28 is not completely evaporated, the residual anesthetic liquid 20 can be accelerated to evaporate again after passing through the narrow channel adjusted by the concentration regulator 31.

### BRIEF DESCRIPTION OF THE DRAWINGS

FIG. 1 is a three-dimensional diagram of a continuous reciprocating drug delivery device according to the present application;
FIG. 2 is a cross-sectional view of a continuous reciprocating drug delivery device according to the present application;
FIG. 3 is a schematic diagram of a base;
FIG. 4 is a schematic diagram of a neutral position of a switching valve;
FIG. 5 is a schematic diagram of drug suction in an upper plunger chamber;
FIG. 6 is a schematic diagram of simultaneous drug suction in the upper plunger chamber and drug pushing in a lower plunger chamber;
FIG. 7 is a schematic diagram of a tank body;
FIG. 8 is a schematic diagram of a top cover;
FIG. 9 is a schematic diagram of an evaporation seat;
FIG. 10 is a schematic diagram of a lower valve plate;
FIG. 11 is a first schematic diagram of an upper valve plate; and
FIG. 12 is a second schematic diagram of an upper valve plate.

### Reference numerals in the accompanying drawings

| | | | | | |
|---|---|---|---|---|---|
| 1. | base | 2. | liquid level column | 3. | tank body |
| 4. | top cover | 5. | drug injection port | 6. | drug cap |
| 7. | first hose | 8. | first solenoid valve | 9. | electronic liquid level gauge |
| 10. | first encoder | 11. | linear motor | 12. | rotary motor |
| 13. | second hose | 14. | rotary motor connecting seat | 15. | third hose |
| 16. | evaporation seat | 17. | drug discharge knob | 18. | pipe plug |
| 19. | first sealing ring | 20. | anesthetic liquid | 21. | sealing gasket |
| 22. | second sealing ring | 23. | connecting sleeve | 24. | end cover |
| 25. | connecting shaft | 26. | thrust bearing | 27. | upper valve plate |
| 28. | lower valve plate | 29. | fresh gas inlet | 30. | plunger rod |
| 31. | concentration regulator | 32. | mixed anesthetic gas outlet | 33 | sealing sleeve |
| 34. | spring retainer ring | 35. | second solenoid valve | 36. | first flat airway port. |
| 37. | second flat airway port | | | | |

### DETAILED DESCRIPTION

The technical solutions provided by the present application are further described below in conjunction with the embodiments.

### Embodiment 1

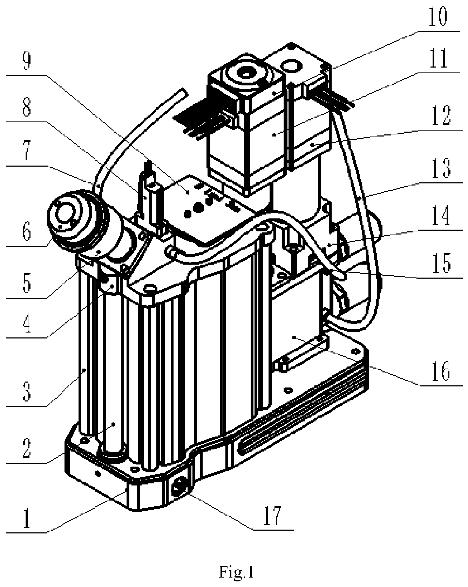

As shown in FIG. 1 to 12, a continuous reciprocating drug delivery device provided by the present application includes: a drug storage tank mechanism, configured to hold anesthetic liquid 20; and an evaporation mechanism, configured to evaporate the anesthetic liquid 20 into a gaseous state to be proportioned with a fresh gas to output a mixed anesthetic gas at a predetermined concentration, the device further includes a plunger pump mechanism, an upper valve plate 27, a lower valve plate 28, and a second solenoid valve 35, where
the evaporation mechanism includes an evaporation seat 16, and the evaporation seat 16 is vertically provided with a deep-hole cavity for use as a total plunger chamber of the plunger pump mechanism;
a sealing sleeve 33 is disposed inside the total plunger chamber and divides the total plunger chamber into an upper plunger chamber and a lower plunger chamber, an upper plunger chamber upper opening and an upper plunger chamber lower opening are provided in a sidewall of the upper plunger chamber, and a lower plunger chamber upper opening and a lower plunger chamber lower opening are provided in a sidewall of the lower plunger chamber;
the plunger pump mechanism is further provided with a plunger rod 30, the plunger rod 30 is connected to an output shaft of a linear motor 11 and moves up and down in the upper plunger chamber and the lower plunger chamber under control of the linear motor 11, the plunger rod 30 includes a plunger rod lower section and a plunger rod upper section, a cross-sectional area of the plunger rod lower section is larger than a cross-sectional area of the plunger rod upper section, a length of the plunger rod lower section is greater than a height of the lower plunger chamber, a bottommost end of the plunger rod lower section passes through the sealing sleeve 33 and remains in the lower plunger chamber, when the plunger rod 30 moves upward, a volume of the upper plunger chamber decreases and a volume of the lower plunger chamber increases, and when the plunger rod 30 moves downward, the volume of the lower plunger chamber decreases and the volume of the upper plunger chamber increases;
the upper valve plate 27 and the lower valve plate 28 are in close contact to form a switching valve, the upper valve plate 27 is driven by a rotary motor 12 to rotate, the upper valve plate 27 and the lower valve plate 28 jointly provide fluid channels separately between a tank body 3 of the drug storage tank mechanism and the upper plunger chamber upper opening, between the upper plunger chamber upper opening and the upper plunger chamber lower opening, between the upper plunger chamber lower opening and an evaporation chamber of the evaporation mechanism, between the tank body 3 and the lower plunger chamber upper opening, between the lower plunger chamber upper opening and the lower plunger chamber lower opening, and/or between the lower plunger chamber lower opening and the evaporation chamber of the evaporation mechanism; and
the lower plunger chamber upper opening is further selectively in communication with the tank body 3 through the second solenoid valve 35.

An outer ring of the sealing sleeve 33 is fixedly connected to an inner wall of the total plunger chamber through a sealing ring, and an inner ring is in close contact with the plunger rod 30 through a sealing ring; and the linear motor 11 is equipped with a first encoder 10 configured to feed back a movement distance of the output shaft of the linear motor 11 in real time to achieve precise control of movement of the plunger rod 30.

An upper end of the evaporation seat 16 is provided with a circular edge-cut groove, a bottom of the groove is provided with a first flat airway port 36 and a second flat airway port 37, the first flat airway port 36 is in communication with a fresh gas inlet 29, the second flat airway port 37 is in communication with a mixed anesthetic gas outlet 32, an evaporation seat boss is disposed between the first flat airway port 36 and the second flat airway port 37, and a hole X'1, a hole X'2, a hole X'3, a hole X'4, and a hole X'5 are circumferentially distributed near an inner edge of the bottom of the circular edge-cut groove, where
the hole X'1 is connected to the tank body 3 of the drug storage tank mechanism through a first internal pipe of the evaporation seat 16;
the hole X'2 is in communication with the upper plunger chamber upper opening through a second internal pipe of the evaporation seat 16;
the hole X'3 is in communication with the upper plunger chamber lower opening through a third internal pipe of the evaporation seat 16;
the hole X'4 is in communication with the lower plunger chamber upper opening through a fourth internal pipe of the evaporation seat 16;
the hole X'5 is in communication with the lower plunger chamber lower opening through a fifth internal pipe of the evaporation seat 16;
the lower valve plate 28 is provided with a circular edge-cut boss corresponding to the circular edge-cut groove of the evaporation seat 16 for blind insertion and positioning between the lower valve plate 28 and the evaporation seat 16, the circular edge-cut boss is further provided with a hole X1, a hole X2, a hole X3, a hole X4, and a hole X5 that correspond one-to-one to the hole X'1, the hole X'2, the hole X'3, the hole X'4, and the hole X'5, respectively, the lower valve plate 28 is provided with a lower valve plate groove, the lower valve plate groove covers the first flat airway port 36, the second flat airway port 37, and the evaporation seat boss, and a gap exists between a bottom surface of the lower valve plate groove and a top surface of the evaporation seat boss, the gap between the bottom surface of the lower valve plate groove and the top surface of the evaporation seat boss is used as the evaporation chamber, and a plurality of through holes are further arranged in an arc at a bottom of the lower valve plate groove; and
the upper valve plate 27 is driven by the rotary motor 12 to rotate, the upper valve plate 27 is symmetrically provided with a first I-shaped groove and a second I-shaped groove, an inner-layer groove and an outer-layer groove of each of the first I-shaped groove and the second I-shaped groove are both arc-shaped, and centers of the inner-layer groove and the outer-layer groove are connected by a straight groove, radians of the outer-layer grooves of the first I-shaped groove and the second I-shaped groove are the same as circumferential radians between the hole X1, the hole X2, the hole X3, the hole X4, and the hole X5, and radians of the inner-layer grooves of the first I-shaped groove and the second I-shaped groove are the same as an arrangement radian of the through holes.

When the outer-layer groove of the first I-shaped groove covers the hole X1 and the hole X2, the first I-shaped groove provides a fluid channel between the hole X1 and the hole X2, and the anesthetic liquid 20 flows from the tank body 3 of the drug storage tank mechanism into the upper plunger chamber of a first plunger pump set sequentially through the hole Y1, the first internal pipe of the evaporation seat 16, the hole X'1, the hole X1, the first I-shaped groove, the hole X2, the hole X'2, the second internal pipe of the evaporation seat 16, and the upper plunger chamber upper opening;
when the outer-layer groove of the first I-shaped groove covers the hole X2 and the hole X3, the first I-shaped groove provides a fluid channel between the hole X2 and the hole X3;
when the outer-layer groove of the first I-shaped groove covers the hole X3 and the inner-layer groove of the first I-shaped groove covers the through holes of the lower valve plate 28, the first I-shaped groove provides a fluid channel between the hole X3 and the through holes, and the anesthetic liquid 20 in the upper plunger chamber flows into the evaporation chamber sequentially through the upper plunger chamber lower opening, the third internal pipe of the evaporation seat 16, the hole X'3, the hole X3, the first I-shaped groove, and the through holes;
when the outer-layer groove of the second I-shaped groove covers the hole X1 and the hole X4, the second I-shaped groove provides a fluid channel between the hole X1 and the hole X4 , and the anesthetic liquid 20 flows from the tank body 3 of the drug storage tank mechanism into the lower plunger chamber sequentially through the hole Y1, the first internal pipe of the evaporation seat 16, the hole X'1, the hole X1, the second I-shaped groove, the hole X4, the hole X'4, the fourth internal pipe of the evaporation seat 16, and the lower plunger chamber upper opening;
when the outer-layer groove of the second I-shaped groove covers the hole X4 and the hole X5, the second I-shaped groove provides a fluid channel between the hole X4 and the hole X5; and
when the outer-layer groove of the second I-shaped groove covers the hole X5 and the inner-layer groove of the second I-shaped groove covers the through holes of the lower valve plate 28, the second I-shaped groove provides a fluid channel between the hole X5 and the through holes, and the anesthetic liquid 20 in the lower plunger chamber flows into the evaporation chamber sequentially through the lower plunger chamber lower opening, the fifth internal pipe of the evaporation seat 16, the hole X'5, the hole X5, the second I-shaped groove, and the through holes.

The first flat airway port 36 is in communication with the fresh gas inlet 29, and the second flat airway port 37 is connected to the mixed anesthetic gas outlet 32; and the anesthetic liquid 20 in the upper plunger chamber or the lower plunger chamber flows into the gap between the lower valve plate groove and the evaporation seat boss through the through holes and evaporates, and the evaporated anesthetic gas and the fresh gas flowing in through the fresh gas inlet 29 are mixed and output through the mixed anesthetic gas outlet 32.

A concentration regulator 31 is disposed between the second flat airway port 37 and the mixed anesthetic gas outlet 32 and configured to adjust a cross-sectional area of a fluid channel between the second flat airway port 37 and the mixed anesthetic gas outlet 32.

The upper valve plate 27, a thrust bearing 26, a connecting shaft 25, a spring, a rotary motor connecting seat 14, the rotary motor 12, and a second encoder are sequentially disposed above the lower valve plate 28, where
the spring is clamped between the rotary motor connecting seat 14 and the thrust bearing 26, a compression force of the spring is sequentially transmitted to the thrust bearing 26, the upper valve plate 27, and the lower valve plate 28 to enable the upper valve plate 27 to be in close contact with the lower valve plate 28;
the upper valve plate 27 is coupled to the connecting shaft 25, and a motor shaft of the rotary motor drives the upper valve plate 27 to rotate through the connecting shaft 25 to achieve transmission of torque; and
the second encoder is configured to position a rotation angle of the upper valve plate 27.

The drug storage tank mechanism includes the tank body 3, a liquid level column 2, an electronic liquid level gauge 9, a pressure sensor, and a first solenoid valve 8, where
the tank body 3 includes a drug injection port 5 configured to inject the anesthetic liquid 20 and a drug discharge port configured to discharge the anesthetic liquid 20 outside, the drug injection port 5 is provided with a drug cap 6, and opening and closing of the drug discharge port are controlled by a drug discharge knob 17;
the liquid level column 2 is in communication with the tank body 3 and configured to manually observe a liquid level height of the tank body 3;
the electronic liquid level gauge 9 is configured to monitor the liquid level height of the tank body 3 in real time;
the pressure sensor is configured to monitor pressure inside the tank body 3 in real time; and
the first solenoid valve 8 is configured to control communication between the tank body 3 and a fresh gas inlet 29.

The following, in conjunction with the accompanying drawings, specifically demonstrates a continuous reciprocating drug delivery device provided by the present application and a working process thereof:
The continuous reciprocating drug delivery device provided in this embodiment includes:
This embodiment includes two major parts: a drug storage tank mechanism and an evaporation module.

As shown in FIG. 1 to FIG. 2, the drug storage tank mechanism includes a base 1, a liquid level column 2, a tank body 3, a top cover 4, a drug injection port 5, a drug cap 6, a first hose 7, a first solenoid valve 8, an electronic liquid level gauge 9, a drug discharge knob 17, a pipe plug 18, a first sealing ring 19, a sealing gasket 21, and a second sealing ring 22.

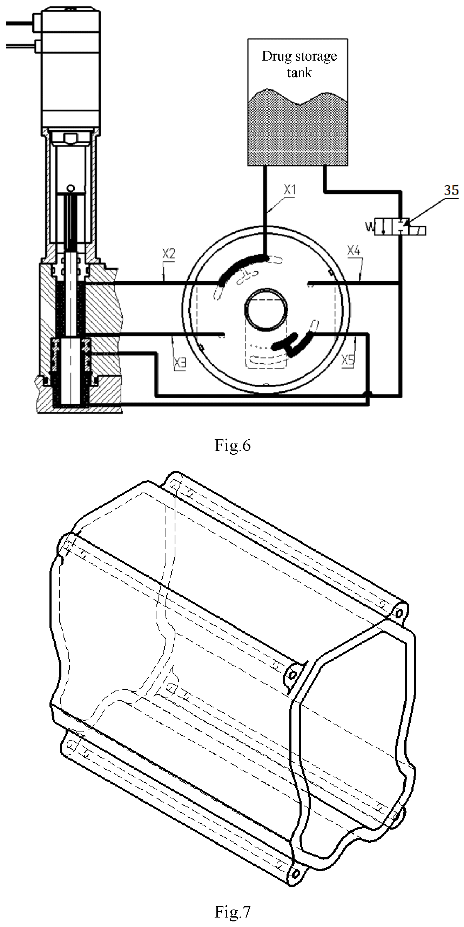

As shown in the schematic diagram of the tank body in FIG. 7, the tank body 3 is made by cutting and processing a formed aluminum profile. There is an arc-shaped groove in the front of the tank body 3, and arc-shaped grooves on the left and right sides of the tank body 3, which are convenient for grasping by hand. There are four threaded holes at each of two ends around the tank body 3.

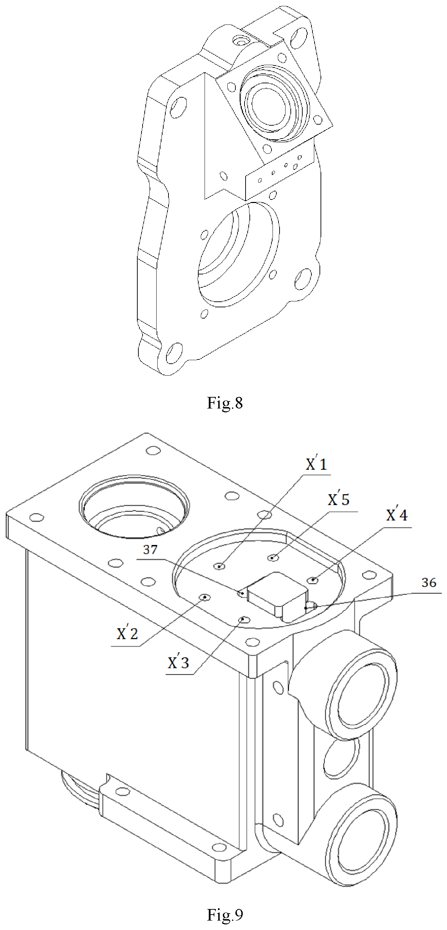

As shown in the schematic diagram of the top cover in FIG. 8, a cylindrical stepped hole is opened on the lower side in the front of the top cover 4. The inside of the cylindrical stepped hole is connected to the inside of the top cover 4 through a small hole. In the upper part of the top cover 4, a circular stepped hole and a triangular installation seat are respectively opened. Four stepped holes are opened on its periphery for screw assembly. A circle of sealing groove is opened on its back.

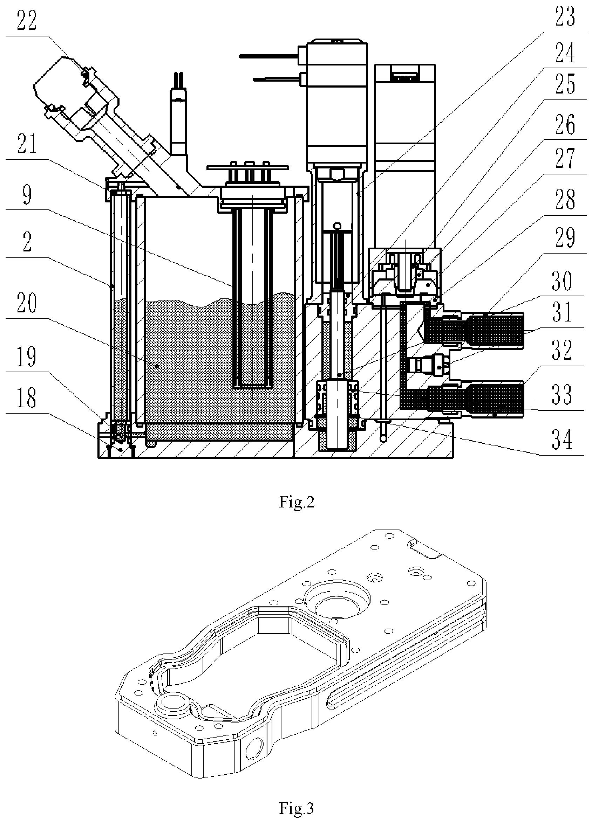

As shown in the schematic diagram of the base in FIG. 3, the front end of the base 1 is provided with a stepped hole with threads. Above the front end, a drug pool groove is provided. On the side of the front end, a drug discharge knob hole is provided, and below it, a drug discharge port is provided. Above the rear end, a plunger pump installation groove is provided. On both sides of the base 1, guide rail grooves are provided.

The specific assembly relationship is as follows: The drug cap 6 is configured with the second sealing ring 22. The tank body 3 is sandwiched between the top cover 4 and the base 1 and is fastened by screws. The electronic liquid level gauge 9 is installed in the circular groove in the upper part of the top cover 4. The electronic liquid level gauge 9 is fixed by screws. The triangular installation seat in the upper part of the top cover 4 is connected to the drug injection port 5 and the first solenoid valve 8 by screws. The triangular installation seat in the upper part of the top cover 4 is also connected to the first hose 7 and the third hose 15 through joints. The first hose 7 is connected to the pressure sensor on the main control board through a pipe, and the pressure sensor monitors the pressure inside the tank body 3 in real time through the first hose 7. The third hose 15 is connected to the evaporation module 16. The on-off of the first solenoid valve 8 can allow the flow of the fresh gas in the fresh gas inlet 29. The fresh gas inlet 29 is the inlet of fresh gas for the evaporation module 16, so that the gas collected by the third hose 15 is the fresh gas entering the drug storage tank. The drug discharge knob 17 is installed in the drug discharge knob hole of the base 1. A conical sealing gasket is installed at the front end of the drug discharge knob 17, the O-shaped first sealing ring 19 is installed in the middle, and the rear end is threaded. The anesthetic in the drug storage tank can be discharged through the drug discharge port below the base 1 by adjusting the drug discharge knob 17.

The evaporation module includes the first encoder 10, the linear motor 11, the rotary motor 12, a second hose 13, the rotary motor connecting seat 14, the evaporation seat 16, the base 1, a connecting sleeve 23, an end cover 24, the connecting shaft 25, the thrust bearing 26, the upper valve plate 27, the lower valve plate 28, the fresh gas inlet 29, the plunger rod 30, the concentration regulator 31, the mixed anesthetic gas outlet 32, the sealing sleeve 33, a spring retainer ring 34, the second solenoid valve 35, a first flat airway port 36, and a second flat airway port 37.

As shown in the schematic diagram of the evaporation seat in FIG. 9, a stepped through hole is opened at an end of the evaporation seat 16, which serves as the plunger cavity of the plunger pump. The sealing sleeve 33 and the plunger rod 30 are placed in the cavity of the plunger pump. The inner ring of the sealing sleeve 33 is connected to the plunger rod 30 by a sealing ring, and the outer ring of the sealing sleeve 33 is connected to the stepped hole of the cavity of the evaporation seat 16 through a sealing ring. The plunger rod 30 is connected to an output shaft of the linear motor 11. The linear motor 11 is connected to the evaporation seat 16 by a linear motor connecting seat through screws. The telescopic movement of the plunger rod 30 is powered by the linear motor 11. A first encoder 10 is equipped at the top of the linear motor 11. The movement distance of the output shaft of the linear motor 11 can be fed back in real time through the first encoder 10, so as to precisely control the movement of the plunger rod 30. As shown in FIG. 2, the plunger rod 30 and the sealing sleeve 33 divide the original cavity into two independent cavities. The volumes of the upper and lower cavities can be changed in real time by controlling the vertical movement of the plunger rod 30 through the linear motor 11. The change in volume can realize the functions of drug suction and drug pushing in the upper and lower cavities. The above-mentioned sealing sleeve 33, plunger rod 30, linear motor 11, evaporation seat 16, connecting sleeve 23, end cover 24, etc. constitute the plunger pump. The plunger pump is installed vertically, and drug inlet and drug outlet nozzles are opened in the upper and lower ends of the upper and lower cavities.

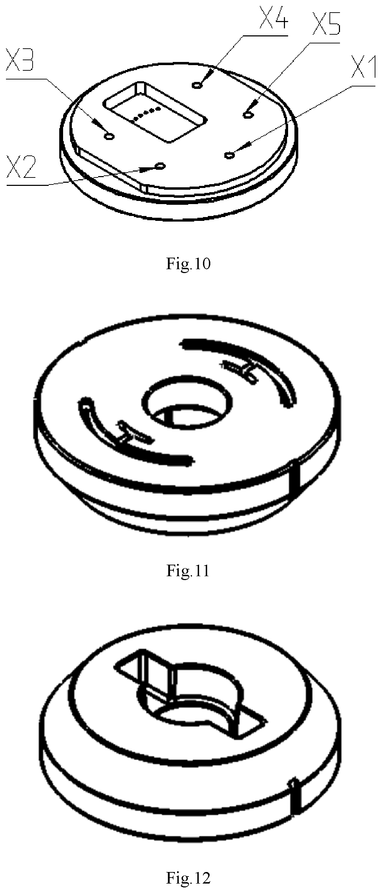

The front end of the evaporation seat 16 is provided with a circular edge-cut groove, and the bottom of the groove is provided with two flat airway holes and five small holes. The first flat airway port 36 is in communication with the fresh gas inlet 29, and the second flat airway port 37 is in communication with the mixed anesthetic gas outlet 32. There is a square boss between the first flat airway port 36 and the second flat airway port 37. This boss is inserted into the lower valve plate 28 (see the schematic diagram of the lower valve plate in FIG. 10) and a certain spacing is left between the boss and a row of small holes at the bottom of the groove of the lower valve plate 28. During the evaporation process of the anesthetic, the anesthetic flows from the small holes to the space between the boss and the evaporation seat 16. The fresh gas then enters from the fresh gas inlet 29 and is mixed with the anesthetic through the first flat airway port 36. Since the spacing between the boss and the small holes of the lower valve plate 28 is relatively small, the flow velocity of the gas flowing here is very high, thereby accelerating the evaporation rate of the anesthetic. After the fresh gas is mixed with the anesthetic, the mixed gas passes through the second flat airway port 37 to reach the mixed anesthetic gas outlet 32, thereby realizing the rapid evaporation of the anesthetic in the vaporizer.

The concentration regulator 31 is installed between the O₂ flat airway hole in the evaporation seat 16 and the mixed anesthetic gas outlet 32. The cross-sectional area of the fluid channel between the second flat airway port 37 and the mixed anesthetic gas outlet 32 can be adjusted by adjusting the concentration regulator 31. As the concentration regulator 31 squeezes the fluid channel, the cross-sectional area of the fluid channel decreases. The concentration regulator 31 adjusts the flow velocity in the fluid channel by adjusting the cross-sectional area of the fluid channel. When the cross-sectional area decreases, that is, after the fluid channel becomes narrow, the flow velocity increases again. If the anesthetic liquid 20 flowing out of the small holes of the lower valve plate 28 is not completely evaporated, the residual anesthetic liquid 20 can be accelerated to evaporate again after passing through the narrow channel adjusted by the concentration regulator 31.

Regarding the installation of the switching valve:
1. The lower valve plate 28 is connected and installed to the evaporation seat 16 by a sealing gasket. Moreover, the holes X'1, X'2 , X'3 , X'4 , and X'5 shown in the schematic diagram of the evaporation seat in FIG. 9 correspond one-to-one to the holes X1, X2, X3, X4, and X5 shown in the schematic diagram of the lower valve plate in FIG. 10. The connecting parts of the evaporation seat 16 and the lower valve plate 28 are both provided with chamfers, which can achieve the function of blind insertion and positioning. In this way, the above-mentioned five small holes are also blind-mated and aligned.

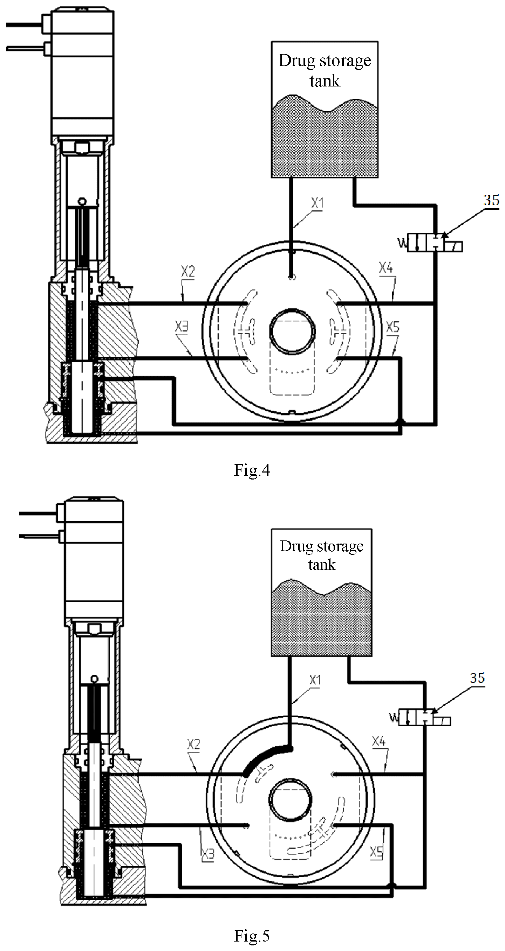

As shown in FIG. 4 to FIG. 6, the connection of the five small holes of the above-mentioned evaporation seat 16 is described as follows:
the hole X'1 is in communication with the tank body 3 of the drug storage tank mechanism through an internal pipe of the evaporation seat 16;
the hole X'2 is in communication with the upper plunger chamber upper opening through an internal pipe of the evaporation seat 16;
the hole X'3 is in communication with the upper plunger chamber lower opening through an internal pipe of the evaporation seat 16;
the hole X'4 is in communication with the lower plunger chamber upper opening through an internal pipe of the evaporation seat 16;
the hole X'5 is in communication with the lower plunger chamber lower opening through an internal pipe of the evaporation seat 16;
The lower plunger chamber upper opening is further selectively connected to the drug tank 3 through the second solenoid valve 35. The second solenoid valve 35 controls the on-off of the pipe between the drug tank and the lower plunger chamber upper opening.

2. As shown in FIG. 2, the upper valve plate 27, the thrust bearing 26, the connecting shaft 25, the spring, the rotary motor connecting seat 14, the rotary motor 12, and the second encoder are sequentially connected above the lower valve plate 28. Specifically, the spring is clamped between the rotary motor connecting seat 14 and the thrust bearing 26, a compression force of the spring is sequentially transmitted to the thrust bearing 26, the upper valve plate 27, and the lower valve plate 28 to enable the upper valve plate 27 to be in close contact with the lower valve plate 28 to achieve sealing. As shown in FIG. 11, a side of the upper valve plate 27 is provided with an I-shaped groove. The inner and outer layers of the I-shaped groove are arc-shaped grooves, and the centers of the inner and outer arc-shaped grooves are connected by a straight groove. The opening and closing of the corresponding valve ports of the lower valve plate 28 can be realized by rotating the upper valve plate 27. For details, refer to the following specific working process. As shown in FIG. 12, two straight grooves are formed in the sidewall of the upper valve plate 27. The outer contour of the connecting shaft 25 has a matching straight flange, which can cooperate with the upper valve plate to realize the transmission of torque. The inner hole of the connecting shaft 25 is matched with the motor shaft. The upper valve plate 27 can be rotated at any angle by controlling the rotary motor 12. The function of the second encoder is to realize the precise positioning of the upper valve plate 27.

The specific working process of the above-mentioned continuous reciprocating drug delivery device is described as follows:

### 1. Drug loading:

The drug cap 6 is opened, and anesthetic is injected into the drug storage tank through the drug injection port 5. The liquid level in the tank can be manually observed through the liquid level column 2. In addition, the electronic liquid level gauge 9 can monitor the liquid level height in real time. After the anesthetic is filled, the drug cap 6 is screwed on.

### 2. Initialization:

The left and right plungers move to the bottommost end, and the switching valve returns to the 0 position, as shown in the schematic diagram of the neutral position of the switching valve in FIG. 4.

3. Gas bubble exhausting: FIG. 5 is a schematic diagram of drug suction in an upper plunger chamber:
After the anesthetic is introduced, there is a large amount of air in the pipes of the evaporation seat 16 and the components thereof. Gas in the pipes needs to be removed to ensure that the plunger pump pushes out only the anesthetic liquid during operation. The specific working process is as follows: The second solenoid valve 35 is controlled to be in the connected state, and the rotary motor 12 is controlled to rotate clockwise by a certain angle. The arc-shaped groove of the upper valve plate 27 connects X1 and X2, and the remaining nozzles are in a closed state. In this case, the upper plunger chamber upper opening of the plunger pump is in communication with the drug storage tank through the hole X1 and the hole X2, and the lower plunger chamber upper opening is in communication with the drug storage tank through the second solenoid valve 35 in a connected state.

The vertical movement of the plunger rod 30 of the plunger pump mechanism is controlled. When the plunger rod 30 moves upward, the volume of the upper plunger chamber decreases. At the same time, air in the upper plunger chamber is squeezed into the drug storage tank. At the same time, the volume of the lower plunger chamber increases, and the liquid drug in the drug storage tank is sucked into the lower plunger chamber. The liquid drug drops to the bottom of the lower plunger chamber by its own weight. When the plunger rod 30 moves downward, the volume of the upper plunger chamber increases. The liquid drug in the drug storage tank flows into the upper plunger chamber through the switching valve and then drops to the bottom of the upper plunger chamber by its own weight. In the case, the volume of the lower plunger chamber decreases. The upper part of the lower plunger chamber holds gas, and the gas is squeezed into the drug storage tank through the second solenoid valve 35. Through the above action steps, the upper plunger chamber and the lower plunger chamber of the plunger pump can be filled with liquid drug, and at the same time, the gas is squeezed into the drug storage tank.

### 4. Control of the evaporation process of anesthetic:

After the above Steps 1, 2, and 3 are completed, both the upper and lower plunger chambers of the plunger pump are filled with anesthetic. In this case, the plunger rod 30 is at the bottommost position, the second solenoid valve 35 is closed, and the upper valve plate 27 is in the 0-position state, as shown in the schematic diagram of the neutral position of the switching valve in FIG. 4.

Taking drug suction in the upper plunger chamber and drug pushing in the lower plunger chamber as an example, as shown in FIG. 6, when a low-concentration output is required, the rotary motor 12 drives the upper valve plate 27 to the position shown in FIG. 6, that is, the inner arc-shaped groove on the lower right side of the upper valve plate 27 covers one small hole of the lower valve plate 28. In this case, a certain amount of anesthetic can be pushed out by pushing the plunger rod 30 at a certain speed. If a high-concentration anesthetic is required, the upper valve plate 27 can be driven to continue rotating at a certain angle, so that the inner arc-shaped groove on the lower right side of the upper valve plate 27 covers more small holes. In this way, the anesthetic liquid 20 with a large flow rate can pass through more smoothly. As can be seen from FIG. 6, regardless of how many small holes are covered by the upper valve plate 27, the hole X2 of the plunger pump remains in a state of being in communication with the hole X1, and the hole X3 and the hole X4 are in a closed state. It can be realized by adjusting the second solenoid valve 35 to the closed state that the drug is pushed out from the lower plunger chamber and simultaneously the drug is sucked in the upper plunger chamber.

Conversely, when the upper valve plate 27 rotates in the opposite direction, the actions of pushing out the drug from the upper plunger chamber and sucking in the drug in the lower plunger chamber can also be completed. Therefore, the vertical reciprocating motion of the plunger rod 30 can realize the continuous drug delivery function of the anesthetic.

### 5: End

When surgery is completed and the supply of anesthetic needs to be stopped, the upper valve plate 27 needs to be rotated to the position shown in FIG. 5, and at the same time, the second solenoid valve 35 is in the connected state. The plunger rod 30 is then controlled to move to the bottommost end position, and then the second solenoid valve 35 is closed. Finally, the upper valve plate 27 is rotated to the 0-point position as shown in FIG. 4 to finish the process.

It can be seen from the above detailed description of the present application that:
1. The present application realizes the precise control and rapid response of anesthetic concentration. The electronically controlled vaporizer provided by the present application controls the volume of the anesthetic by controlling the pushing speed of the anesthetic liquid 20, thereby improving the accuracy of the output anesthetic concentration by utilizing the incompressibility of liquid volume. The influence of temperature, air pressure, etc. on the liquid anesthetic can be ignored. The linear motor is used to precisely control the drug-pushing speed of the plunger pump. The output anesthetic is immediately evaporated after being mixed with fresh gas, and therefore a definite anesthetic concentration is obtained. If different concentrations of anesthetic gas at different flow rates are desired, it is only necessary to control the flow rate of the fresh gas and the drug-pushing speed of the plunger pump.
2. The present application only uses one switching valve. The enablement and disablement of flow at the drug inlets and outlets of the two plunger pump assemblies are controlled by controlling the rotation angle of the switching valve in combination with the on-off of the second solenoid valve, so as to realize the drug suction and drug pushing functions of the plunger pumps. When one plunger pump assembly is sucking in drug, the other plunger pump assembly can push out drug simultaneously. Moreover, using only one switching valve results in a small assembly difficulty, fewer points to be sealed, and low costs. Therefore, the structure is simple, which is conducive to processing and production and easy for productization.
3. The present application utilizes a plunger-type plunger pump mechanism, which enables the plunger pump mechanism to be placed vertically, and the plunger chamber is located in the evaporation seat. This is beneficial for product miniaturization. Moreover, the overall structural form is more compact, the weight is lighter, the installation of pipes is reduced, and the reliability is greatly improved.
4. Compared with the piston structure adopted by the existing drug delivery devices, which requires fine machining of the inner hole of the plunger pump cavity, the present application adopts a plunger-type plunger pump mechanism, which performs fine machining on the plunger rod. The outer surface roughness of the plunger rod is required to be relatively high. However, the outer surface of the plunger rod is an outer circle, which is very easy to perform fine machining on, and the processing costs are low.
5. The concentration regulator 31 is installed between the O₂ flat airway hole in the evaporation seat 16 and the mixed anesthetic gas outlet 32. The cross-sectional area of the fluid channel between the second flat airway port 37 and the mixed anesthetic gas outlet 32 can be adjusted by adjusting the concentration regulator 31. As the concentration regulator 31 squeezes the fluid channel, the cross-sectional area of the fluid channel decreases. The concentration regulator 31 adjusts the flow velocity in the fluid channel by adjusting the cross-sectional area of the fluid channel. When the cross-sectional area decreases, that is, after the fluid channel becomes narrow, the flow velocity increases again. If the anesthetic liquid 20 flowing out of the small holes of the lower valve plate 28 is not completely evaporated, the residual anesthetic liquid 20 can be accelerated to evaporate again after passing through the narrow channel adjusted by the concentration regulator 31.

Finally, it should be noted that the foregoing embodiments are merely intended for describing the technical solutions of the present application rather than limiting the present application. Although the present application is described in detail with reference to the embodiments, persons of ordinary skill in the art should understand that they may still make modifications or equivalent replacements to the technical features of the present application without departing from the spirit and scope of the technical solutions of the technical solutions of the present application. These modifications or equivalent replacements shall all fall within the scope of the claims of the present application.

## Claims

1. A continuous reciprocating drug delivery device, comprising: a drug storage tank mechanism, configured to hold anesthetic liquid (20); and an evaporation mechanism, configured to evaporate the anesthetic liquid (20) into a gaseous state to be proportioned with a fresh gas to output a mixed anesthetic gas at a predetermined concentration, **characterized in that** the device further comprises a plunger pump mechanism, an upper valve plate (27), a lower valve plate (28), and a second solenoid valve (35), wherein
the evaporation mechanism comprises an evaporation seat (16), and the evaporation seat (16) is vertically provided with a deep-hole cavity for use as a total plunger chamber of the plunger pump mechanism;
a sealing sleeve (33) is disposed inside the total plunger chamber and divides the total plunger chamber into an upper plunger chamber and a lower plunger chamber, an upper plunger chamber upper opening and an upper plunger chamber lower opening are provided in a sidewall of the upper plunger chamber, and a lower plunger chamber upper opening and a lower plunger chamber lower opening are provided in a sidewall of the lower plunger chamber;
the plunger pump mechanism is further provided with a plunger rod (30), the plunger rod (30) is connected to an output shaft of a linear motor (11) and moves up and down in the upper plunger chamber and the lower plunger chamber under control of the linear motor (11), the plunger rod (30) comprises a plunger rod lower section and a plunger rod upper section, a cross-sectional area of the plunger rod lower section is larger than a cross-sectional area of the plunger rod upper section, a length of the plunger rod lower section is greater than a height of the lower plunger chamber, a bottommost end of the plunger rod lower section passes through the sealing sleeve (33) and remains in the lower plunger chamber, when the plunger rod (30) moves upward, a volume of the upper plunger chamber decreases and a volume of the lower plunger chamber increases, and when the plunger rod (30) moves downward, the volume of the lower plunger chamber decreases and the volume of the upper plunger chamber increases;
the upper valve plate (27) and the lower valve plate (28) are in close contact to form a switching valve, the upper valve plate (27) is driven by a rotary motor (12) to rotate, the upper valve plate (27) and the lower valve plate (28) jointly provide fluid channels separately between a tank body (3) of the drug storage tank mechanism and the upper plunger chamber upper opening, between the upper plunger chamber upper opening and the upper plunger chamber lower opening, between the upper plunger chamber lower opening and an evaporation chamber of the evaporation mechanism, between the tank body (3) and the lower plunger chamber upper opening, between the lower plunger chamber upper opening and the lower plunger chamber lower opening, and/or between the lower plunger chamber lower opening and the evaporation chamber of the evaporation mechanism; and
the lower plunger chamber upper opening is further selectively in communication with the tank body (3) through the second solenoid valve (35).

2. The continuous reciprocating drug delivery device according to claim 1, **characterized in that** an outer ring of the sealing sleeve (33) is fixedly connected to an inner wall of the total plunger chamber through a sealing ring, and an inner ring is in close contact with the plunger rod (30) through a sealing ring; and the linear motor (11) is equipped with a first encoder (10) configured to feed back a movement distance of the output shaft of the linear motor (11) in real time to achieve precise control of movement of the plunger rod (30).

3. The continuous reciprocating drug delivery device according to claim 1, **characterized in that** an upper end of the evaporation seat (16) is provided with a circular edge-cut groove, a bottom of the groove is provided with a first flat airway port (36) and a second flat airway port (37), the first flat airway port (36) is in communication with a fresh gas inlet (29), the second flat airway port (37) is in communication with a mixed anesthetic gas outlet (32), an evaporation seat boss is disposed between the first flat airway port (36) and the second flat airway port (37), and a hole X'1 , a hole X'2 , a hole X'3 , a hole X'4 , and a hole X'5 are circumferentially distributed near an inner edge of the bottom of the circular edge-cut groove, wherein
the hole X'1 is connected to the tank body (3) of the drug storage tank mechanism through a first internal pipe of the evaporation seat (16);
the hole X'2 is in communication with the upper plunger chamber upper opening through a second internal pipe of the evaporation seat (16);
the hole X'3 is in communication with the upper plunger chamber lower opening through a third internal pipe of the evaporation seat (16);
the hole X'4 is in communication with the lower plunger chamber upper opening through a fourth internal pipe of the evaporation seat (16);
the hole X'5 is in communication with the lower plunger chamber lower opening through a fifth internal pipe of the evaporation seat (16);
the lower valve plate (28) is provided with a circular edge-cut boss corresponding to the circular edge-cut groove of the evaporation seat (16) for blind insertion and positioning between the lower valve plate (28) and the evaporation seat (16), the circular edge-cut boss is further provided with a hole X1, a hole X2, a hole X3, a hole X4, and a hole X5 that correspond one-to-one to the hole X'1, the hole X'2, the hole X'3, the hole X'4, and the hole X'5, respectively, the lower valve plate (28) is provided with a lower valve plate groove, the lower valve plate groove covers the first flat airway port (36), the second flat airway port (37), and the evaporation seat boss, and a gap exists between a bottom surface of the lower valve plate groove and a top surface of the evaporation seat boss, the gap between the bottom surface of the lower valve plate groove and the top surface of the evaporation seat boss is used as the evaporation chamber, and a plurality of through holes are further arranged in an arc at a bottom of the lower valve plate groove; and
the upper valve plate (27) is driven by the rotary motor (12) to rotate, the upper valve plate (27) is symmetrically provided with a first I-shaped groove and a second I-shaped groove, an inner-layer groove and an outer-layer groove of each of the first I-shaped groove and the second I-shaped groove are both arc-shaped, and centers of the inner-layer groove and the outer-layer groove are connected by a straight groove, radians of the outer-layer grooves of the first I-shaped groove and the second I-shaped groove are the same as circumferential radians between the hole X1, the hole X2, the hole X3, the hole X4, and the hole X5, and radians of the inner-layer grooves of the first I-shaped groove and the second I-shaped groove are the same as an arrangement radian of the through holes.

4. The continuous reciprocating drug delivery device according to claim 3, **characterized in that** when the outer-layer groove of the first I-shaped groove covers the hole X1 and the hole X2, the first I-shaped groove provides a fluid channel between the hole X1 and the hole X2, and the anesthetic liquid (20) flows from the tank body (3) of the drug storage tank mechanism into the upper plunger chamber of a first plunger pump set sequentially through the hole Y1, the first internal pipe of the evaporation seat (16), the hole X'1, the hole X1, the first I-shaped groove, the hole X2, the hole X'2, the second internal pipe of the evaporation seat (16), and the upper plunger chamber upper opening;
when the outer-layer groove of the first I-shaped groove covers the hole X2 and the hole X3, the first I-shaped groove provides a fluid channel between the hole X2 and the hole X3; when the outer-layer groove of the first I-shaped groove covers the hole X3 and the inner-layer groove of the first I-shaped groove covers the through holes of the lower valve plate (28), the first I-shaped groove provides a fluid channel between the hole X3 and the through holes, and the anesthetic liquid (20) in the upper plunger chamber flows into the evaporation chamber sequentially through the upper plunger chamber lower opening, the third internal pipe of the evaporation seat (16), the hole X'3, the hole X3, the first I-shaped groove, and the through holes;
when the outer-layer groove of the second I-shaped groove covers the hole X1 and the hole X4, the second I-shaped groove provides a fluid channel between the hole X1 and the hole X4, and the anesthetic liquid (20) flows from the tank body (3) of the drug storage tank mechanism into the lower plunger chamber sequentially through the hole Y1, the first internal pipe of the evaporation seat (16), the hole X'1, the hole X1, the second I-shaped groove, the hole X4, the hole X'4, the fourth internal pipe of the evaporation seat (16), and the lower plunger chamber upper opening;
when the outer-layer groove of the second I-shaped groove covers the hole X4 and the hole X5, the second I-shaped groove provides a fluid channel between the hole X4 and the hole X5; and
when the outer-layer groove of the second I-shaped groove covers the hole X5 and the inner-layer groove of the second I-shaped groove covers the through holes of the lower valve plate (28), the second I-shaped groove provides a fluid channel between the hole X5 and the through holes, and the anesthetic liquid (20) in the lower plunger chamber flows into the evaporation chamber sequentially through the lower plunger chamber lower opening, the fifth internal pipe of the evaporation seat (16), the hole X'5, the hole X5, the second I-shaped groove, and the through holes.

5. The continuous reciprocating drug delivery device according to claim 3, **characterized in that** the first flat airway port (36) is in communication with the fresh gas inlet (29), and the second flat airway port (37) is connected to the mixed anesthetic gas outlet (32); and the anesthetic liquid (20) in the upper plunger chamber or the lower plunger chamber flows into the gap between the lower valve plate groove and the evaporation seat boss through the through holes and evaporates, and the evaporated anesthetic gas and the fresh gas flowing in through the fresh gas inlet (29) are mixed and output through the mixed anesthetic gas outlet (32).

6. The continuous reciprocating drug delivery device according to claim 3, **characterized in that** a concentration regulator (31) is disposed between the second flat airway port (37) and the mixed anesthetic gas outlet (32) and configured to adjust a cross-sectional area of a fluid channel between the second flat airway port (37) and the mixed anesthetic gas outlet (32).

7. The continuous reciprocating drug delivery device according to claim 3, **characterized in that** the upper valve plate (27), a thrust bearing (26), a connecting shaft (25), a spring, a rotary motor connecting seat (14), the rotary motor (12), and a second encoder are sequentially disposed above the lower valve plate (28), wherein
the spring is clamped between the rotary motor connecting seat (14) and the thrust bearing (26), a compression force of the spring is sequentially transmitted to the thrust bearing (26), the upper valve plate (27), and the lower valve plate (28) to enable the upper valve plate (27) to be in close contact with the lower valve plate (28);
the upper valve plate (27) is coupled to the connecting shaft (25), and a motor shaft of the rotary motor drives the upper valve plate (27) to rotate through the connecting shaft (25) to achieve transmission of torque; and
the second encoder is configured to position a rotation angle of the upper valve plate (27).

8. The continuous reciprocating drug delivery device according to claim 1, **characterized in that** the drug storage tank mechanism comprises the tank body (3), a liquid level column (2), an electronic liquid level gauge (9), a pressure sensor, and a first solenoid valve (8), wherein
the tank body (3) comprises a drug injection port (5) configured to inject the anesthetic liquid (20) and a drug discharge port configured to discharge the anesthetic liquid (20) outside, the drug injection port (5) is provided with a drug cap (6), and opening and closing of the drug discharge port are controlled by a drug discharge knob (17);
the liquid level column (2) is in communication with the tank body (3) and configured to manually observe a liquid level height of the tank body (3);
the electronic liquid level gauge (9) is configured to monitor the liquid level height of the tank body (3) in real time;
the pressure sensor is configured to monitor pressure inside the tank body (3) in real time; and the first solenoid valve (8) is configured to control communication between the tank body (3) and a fresh gas inlet (29).
